# EUROPEAN PATENT APPLICATION

(11) **EP 2 163 615 A1**
(43) Date of publication of application: **17.03.2010**
(21) Application number: 08015750.6
(22) Date of filing: 06.09.2008
(51) Int. Cl.: C12N 9/04, C12N 15/82

(54) **Method for producing eukaryotic organisms with enhanced pathogen resistance and/or resistance to stress and eukaryotic transgenic organisms with enhanced pathogen resistance and/or resistance to stress**

(71) Applicant: Westfälische Wilhelms-Universität Münster, 48149 Münster (DE)
(72) Inventor: von Schaewen, Antje, Prof. Dr., 48161 Münster (DE); Scharte, Judith, 48155 Münster (DE); Tjaden, Zeina, 26121 Oldenburg (DE); Schön, Hardy, 48155 Münster (DE); Becker, Olessja, 59065 Hamm (DE); Fischer, Kerstin, 48159 Münster (DE)
(74) Representative: Stoppkotte, Cornelia

(57) **Abstract**

The present invention relates to a method for producing eukaryotic organisms having enhanced pathogen resistance and/or resistance to stress, comprising the step of expressing in the cytosol of said organisms a glucose-6-phosphate dehydrogenase with increased NADPH tolerance compared to the endogenous cytosolic glucose-6-phosphate dehydrogenase. Furthermore, the present invention relates to the respective eukaryotic transgenic organisms showing enhanced pathogen resistance and/or resistance to stress.

SEQUENCE LISTING

<110> Westfälische Wilhelms-Universität Münster

<120> Method for producing eukaryotic organisms with enhanced pathogen
resistance and/or resistance to stress and eukaryotic transgenic
organisms with enhanced pathogen resistance and/or resistance to
stress

<130> P1050EP00

<160> 5

<170> PatentIn version 3.3

<210> 1
<211> 48
<212> DNA
<213> Artificial sequence

<220>
<223> Primer

<220>
<221> misc_feature
<222> (1)..(6)
<223> n is a, c, g, or t

<400> 1
nnnnnnggat ccaagatggt tgtcgtgcaa gatggatcag tagccacc 48

<210> 2
<211> 42
<212> DNA
<213> Artificial sequence

<220>
<223> Primer

<220>
<221> misc_feature
<222> (1)..(6)
<223> n is a, c, g, or t

<400> 2
nnnnnngtcg actcactgat caagacttag gtctccccat tg 42

<210> 3
<211> 33
<212> DNA
<213> Artificial sequence

<220>
<223> Primer


<220>
<221> misc_feature
<222> (1)..(3)
<223> n is a, c, g, or t

<400> 3
nnnccatggt tgtcgtgcaa gatggatcag tag 33


<210> 4
<211> 34
<212> DNA
<213> Artificial sequence

<220>
<223> Primer

<400> 4
caccgtcgac aatatgaagg ctataaggat gacc 34


<210> 5
<211> 34
<212> DNA
<213> Artificial sequence

<220>
<223> Primer

<400> 5
ggatcctata tgacaggtct aattcacttt gaac 34

## Description

The present invention relates to a method for producing eukaryotic organisms having enhanced pathogen resistance and/or resistance to stress as well as the respective eukaryotic transgenic organisms showing enhanced pathogen resistance and/or resistance to stress.

Organisms use a wide range of mechanisms to resist pathogens and stress, which are usually complicated and difficult to control. However, as organisms with enhanced resistance are desirable in particular in the fields of agricultural crops, useful plants or domestic animals, a method for enhancing pathogen resistance and/or resistance to stress would be of great economic interest.

Glucose-6-phosphate dehydrogenase (G6PDH, EC 1.1.1.49) catalyses the first committed step of the oxidative pentose phosphate pathway (OPPP), an important catabolic route for the provision of NADPH and sugar phosphates. G6PDH is present in every eukaryotic organism and is normally contained in the cytosol as well as in various organelles. In plants, G6PDH activity is present at least in both cytosol and plastids, and possibly also in peroxisomes. The chloroplastic enzyme is known to be reductively inactivated in the light by the ferredoxin/thioredoxin system to avoid futile interactions with the Calvin cycle during photosynthesis. The main role of G6PDH in plastids during the night or in heterotrophic tissues is the supply of reducing equivalents in the form of NADPH required for multiple anabolic reactions (e.g. amino acid or fatty acid synthesis). Moreover, sugar-phosphate intermediates that serve as precursors of nucleotides and secondary plant products are generated.

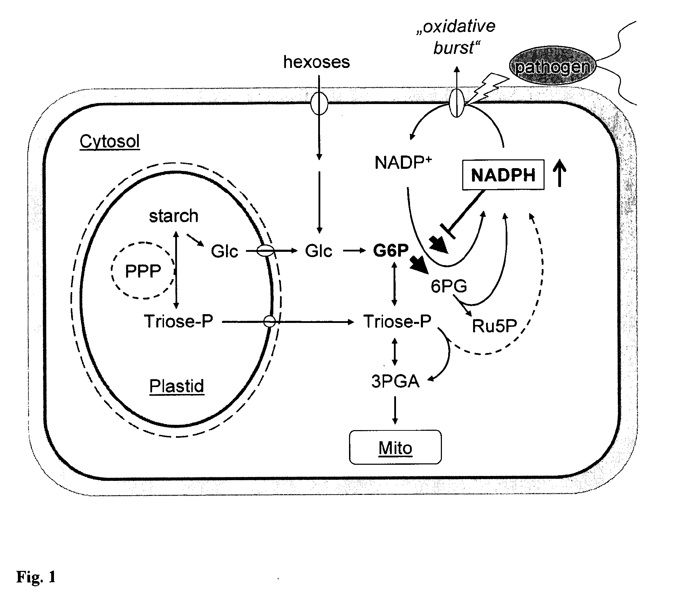

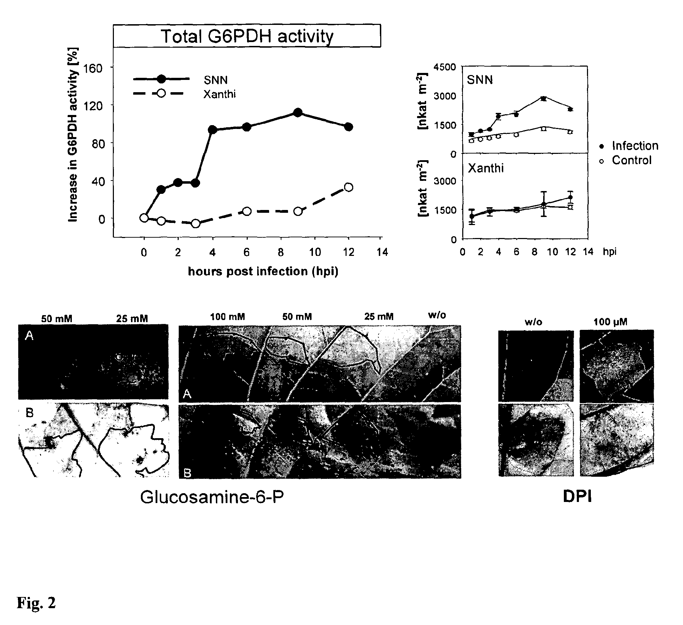

As mentioned above, enzyme reactions of the oxidative pentose phosphate pathway (OPPP) provide reduction power for anabolic biosyntheses in the form of NADPH. This also plays a role during early defence reactions, i.e. upon elicitation of NADPH oxidase at the plasma membrane ("oxidative burst"), an early and evolutionary conserved attempt of eukaryotic cells to interfere with pathogen invasion (see Figs. 1 and 2).

It is an object of the present invention to obtain eukaryotic organisms having enhanced fitness, i.e. enhanced pathogen resistance and/or resistance to stress.

This object is solved by a method according to claim 1 as well as a transgenic organism according to claim 11.

Accordingly, the present invention is directed to a method for producing eukaryotic organisms having enhanced pathogen resistance and/or resistance to stress, comprising the step of:
Expressing in the cytosol of said organism a glucose-6-phosphate dehydrogenase with increased NADPH tolerance compared to the endogenous cytosolic glucose-6-phosphate dehydrogenase.

In this context, increased NADPH tolerance means that the K_{i[NADPH]} of the expressed G6PDH is enhanced compared to the endogenous cytosolic G6PDH.

The present inventors surprisingly found that when they introduced and expressed a glucose-6-phosphate dehydrogenase having increased NADPH tolerance in the cytosol of for example transgenic plants, these organisms showed enhanced pathogen resistance as well as resistance to other stresses. In particular, the replacement of cytosolic G6PDH by a kinetically superior isoenzyme was able to enhance resistance in the progeny of a susceptible plant variety.

In addition to producing complete organisms, the inventive method may also be used to produce only parts of an organism, e.g. organs, tissues or cells. Therefore, for the purpose of the present application the term "organism" is meant to also include parts of an organism as mentioned above. While complete organisms are nonhuman organisms, the parts of an organism also include human parts.

Preferably, the G6PDH with increased NADPH tolerance is an exogenous G6PDH, i.e. an isoenzyme from a different organism. However, if available, it is also possible to introduce and express a non-cytosolic G6PDH with increased NADPH tolerance from the same organism. Furthermore, the G6PDH with increased NADPH tolerance may be a mutated natural, an artificial and/or a genetically engineered modified enzyme.

In this regard, the present inventors found that the improvement in pathogen resistance and resistance to stress increased when the phylogenetic relationship was more distant (Solanaceae versus Arabidopsis G6PDH: 75% identity at the amino acid level).

In a further preferred embodiment of the present invention, the kinetic data of the G6PDH with increased NADPH tolerance fulfil the following relationship: K_{i[NADPH]} > K_{m[NADP+]}, preferably K_{i[NADPH]} ≥ 2 x K_{m[NADP+]}, in particular K_{i[NADPH]} ≥ 5 x K_{m[NADP+]} and most preferred K_{i[NADPH]} ≥ 10 x K_{m[NADP+]}. In this regard, it is particularly preferred to use a genetically engineered enzyme fulfilling the relationship K_{i[NADPH]} ≥ 10 x K_{m[NADP+]}.

In the context of the present specification, K_{i[NADPH]} and K_{m[NADP+]} are empirically determined kinetic enzyme parameters. Methods for determining these parameters are standard methods known to a person skilled in the art. As an example, these parameters may be determined as described in Wendt et al. (2000) for potato G6PDH and in Wakao & Benning (2005) for Arabidopsis isoenzymes.

Furthermore, it is also preferred that the G6PDH with increased NADPH tolerance is an extra-cytoplasmic G6PDH (e.g. plastidic or peroxisomal) that naturally show these kinetic properties. Particularly preferred in this regard is one of the two plastidic P2 isoenzymes (and possibly also the P1 isoform) derived from Arabidopsis, which is described in more detail below.

In another preferred embodiment, the G6PDH with increased NADPH tolerance is overexpressed to reach a concentration in the cytosol of at least twofold (in relation to the concentration of the endogenous enzyme).

It is particularly preferred that the inventive method comprises an additional step of reducing, eliminating or suppressing the activity of the endogenous cytosolic G6PDH. Particularly preferred is an enzyme replacement of the endogenous cytosolic G6PDH by an isoenzyme with increased NADPH tolerance, so that no activity of the endogenous cytosolic G6PDH remains.

Reducing, eliminating or suppressing the activity of endogenous cytosolic G6PDH is preferably carried out by targeted knock-out mutation or gene silencing, co-suppression, antisense or RNA interference, which are all standard methods belonging to the general knowledge of a person skilled in the art.

The eukaryotic organism is preferably selected from plants, animals or fungi, wherein the term "animals" includes vertebrates as well as invertebrates. As already mentioned above, the term "organism" also includes parts of an organism, and in particular also human cell lines.

In case that the eukaryotic organism is a plant, in particular a higher plant species, this plant is preferably selected from the group consisting of Solanaceae, soy bean, maize, rice, Arabidopsis or else, particularly preferred a plant selected from tobacco, tomato, potato, pepper or else.

The present invention also relates to eukaryotic transgenic organisms having enhanced pathogen resistance and/or resistance to stress, **characterized in that** they express in their cytosol a G6PDH with increased NADPH tolerance compared to the endogenous cytosolic enzyme.

These eukaryotic transgenic organisms are obtainable by the inventive method as described above, and they preferable are characterized by one or more of the features described above with respect to the inventive method.

As a preferred embodiment, this invention describes the benefits of enzyme replacement in the cytosol using an antisense approach for eliminating endogenous glucose-6-phosphate dehydrogenase activity combined with expression of an N-terminally truncated plastidial isoenzyme of P2 class, with enhanced K_{i[NADPH]}.

The invention will now be described and explained with respect to the included figures and the following examples. These examples, however, are not intended to limit the scope of the present invention.

The figures show:
Fig. 1: Scheme of the G6PDH "enzyme replacement" strategy. Abbr.: Glc, glucose; G6P, glucose-6-phosphate; Mito, Mitochondrium; NADP⁺/H, nicotinamide-dinucleotide phosphate, oxidized or reduced form; 3PGA, 3-phosphoglycerate; 6PG, 6-phosphogluconate; PPP, pentose-phosphate pathway; Ru5P, ribulose-5-phosphate; Triose-P, triose phosphates DHAP and GA3P (dihydroxyacetone phosphate and glyceraldehyde-3-phosphate). For further explanation see text above.
Fig. 2: G6PDH and NADPH oxidase inhibitors interfere with necrosis formation. Top, G6PDH activity increases dramatically in the resistant tobacco wild type variety SNN after infection with *Phythophthora nicotianae* (*P. nicotianae*; see also Scharte et al., 2005) compared to susceptible wild type Xanthi plants. This suggested a key role for G6PDH in successful plant defence. Bottom, Interference of Glucosamin-6-P (G6PDH inhibitor, infiltrated area marked red) and Diphenylene iodonium (DPI, NADPH oxidase inhibitor) with defence-induced H₂O₂ production (visualized by DAB-staining - after removal of chlorophyll) and the formation of hypersensitive lesions indicate that efficient plant defence depends upon NADPH availability in the cytosol, stemming mostly from the OPPP. Abbr.: w/o, without.

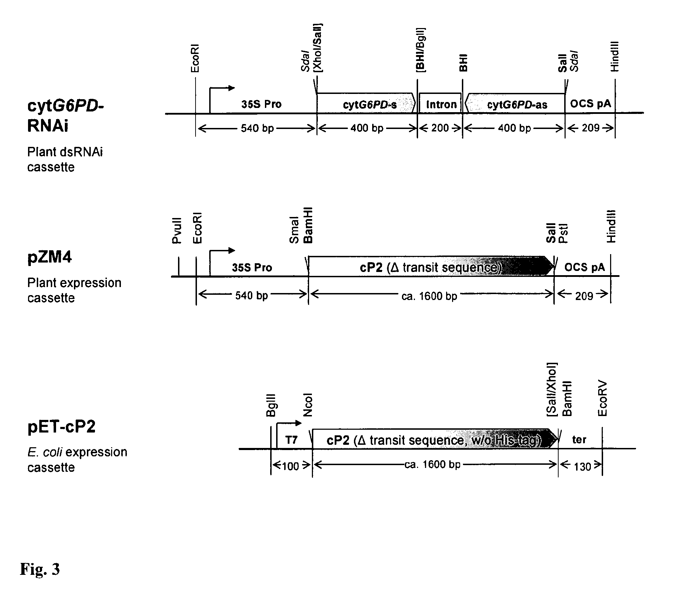

Fig. 3: Construct design for the conducted studies.

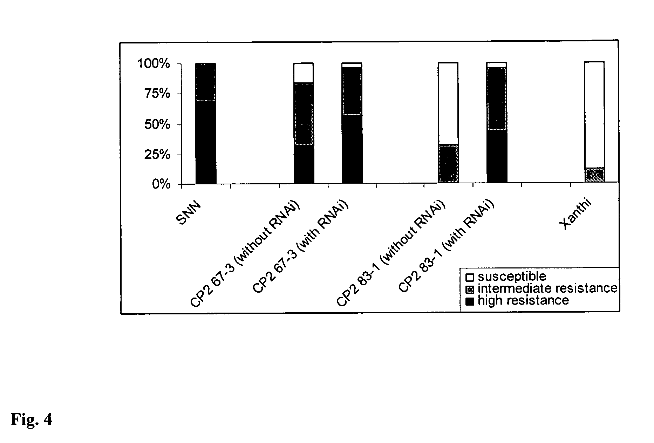

Fig. 4: Disease evaluation of parental cP2 lines versus their corresponding super-transformed RNAi progeny after pathogen challenge with *P. nicotianae*.
Side-by side analysis of weak and strong parental cP2 lines versus their corresponding super-transformed RNAi progeny compared to SNN and Xanthi wild type varieties.
The extent of hypersensitive lesions formed at fifty infiltration sites with zoospores of *P. nicotianae* on 5 plants (three or more independent rounds of infection) was evaluated after 2 days: High resistance, 80-100% lpa (lesions per area); intermediate resistance, 35-80% lpa; susceptible response, 0-10% lpa at infiltration site. The data obtained and illustrated in Fig. 4 are also summarized in Table 1 below:

**Table 1**

| **Variety/trangenic line** | | | |
|---|---|---|---|
| | **Susceptible** | **Intermediate resistance** | **High resistance** |
| SNN (resistant wt) | 0 | 16 | 34 |
| 67-3 (without RNAi) | 8 | 26 | 16 |
| 67-3 (with RNAi) | 2 | 20 | 28 |
| 83-1 (without RNAi) | 34 | 16 | 0 |
| 83-1 (with RNAi) | 2 | 26 | 22 |
| Xanthi (susceptible wt) | 44 | 6 | 0 |

### Examples

Tobacco *Nicotiana tabacum* lines of the susceptible variety Xanthi (Way et al., 2002) were engineered to replace endogenous cytsolic G6PDH activity by an N-terminally truncated plastidial isoenzyme with superior biochemical characteristics (see Fig. 3). Transgenic lines expressing *Arabidopsis thaliana G6PD* isoform At1g24280 (P2 class) without transit peptide were additionally transformed with an RNAi construct eliminating expression of endogenous tobacco *G6PD* isoforms in the cytosol. "Enzyme replaced" Xanthi super-transformants reacted strongly (*hypersensitive*) after zoospore infection with *Phytophthora nicotianae* comparable to the natural tobacco wild type variety Samsun NN. Moreover, extent of necrosis formation was independent of the response displayed by the parental lines (weak to intermediate responses, see Fig. 4). This is an example that effective metabolic channelling ensues only when competing enzyme activities are eliminated in the same cellular compartment.

### Example 1: Measurement of G6PDH and specific inhibitor studies.

### Photometric determination of G6PDH activity

Freshly cut leaf discs were frozen in liquid N₂ and ground to a fine powder. Extraction and determination of G6PDH activity was according to the method described by Fickenscher & Scheibe (1986).

### Inhibitor studies

Evidence for the involvement of NADPH oxidase as the source of ROS-formation is provided by the inhibitory effect of the flavoprotein inhibitor diphenylene iodonium (DPI). DPI is a well-known inhibitor of the mammalian neutrophil oxidase and inhibits also plant NAD(P)H oxidases (Pugin et al., 1997 and references cited therein). NADPH oxidase was shown to be the main source for extracellular ROS production (oxidative burst) in plants upon elicitation (Pugin et al., 1997). This leads to the formation of hypersensitive lesions in leaf tissue of resistant varieties after pathogen infection (incompatible interaction).

When tobacco leaves were treated with 25 -100 mM DPI, infection with *P. nicotianae* did not induce the formation of hypersensitive lesions.

The involvement of the oxidative pentose phosphate pathway (OPPP) in the oxidative burst of plants was shown by inhibitor studies with Glucosamine-6 phosphate (Glucosamine-6-P). This is a well-known competitive inhibitor for G6PDH (Glaser and Brown, 1955), the first enzyme of the OPPP, which transforms G6P into 6-phosphogluconolactone. When tobacco leaves are treated with 25 - 50 mM GN6P, infection with *P. nicotianae* did not result in detectable ROS production.

### Example 2: Zoospore infiltration, H₂O₂ detection, and evaluation of leaf necroses formation.

### Oomycete Growth, Zoospore Production, and Inoculation

*Phytophthora nicotianae* van Breda de Haan isolate 1828 (DSMZ, Braunschweig, GER) was cultivated at 24°C on clarified tomato agar as described by von Broembsen and Deacon (1996). Zoospores were produced under aseptic conditions according to von Broembsen and Deacon (1996). Source leaves from 8- to 10-week-old tobacco plants were infiltrated with a suspension containing 500 to 1,000 zoospores µL⁻¹ according to Colas et al. (2001). This zoospore-leaf infiltration assay was chosen to achieve a rapid, synchronized infection start in all parenchymatic cells of the infiltrated leaf area. For mock-inoculation, sterile tap water was infiltrated, and is further referred to as control. To take into account individual plant or developmental variations, samples from control and infection sites were excised from adjacent intercostal areas of the same source leaf. Plant inoculation was always performed at the beginning of the photoperiod.

### Histochemical detection of H₂O₂

Hydrogen peroxide (H₂O₂) accumulation was detected by *in situ* staining with 3,3-diaminobenzidine (DAB) following a modified protocol of Thordal-Christensen et al. (1997). Leaves were placed in DAB solution (1 mg mL⁻¹, pH 3.8) for 6 h. A dark-brown polymerization product formed at sites where DAB reacts with H₂O₂ produced by the tissue. Incubations were stopped and leaf tissue was simultaneously cleared from chlorophyll by boiling in ethanol for 10 min.

### Determination of the extent of hypersensitive lesions

The extent of hypersensitive lesions formed at fifty infiltration sites with zoospores of *Phytophthora nicotianae* on 5 plants (three or more independent rounds of infections) were evaluated after 2 days: High resistance, 80-100% lpa (lesions per area); intermediate resistance, 35-80% lpa; susceptible response, 0-10% lpa at infiltration site.

### Example 3: Cloning strategy of cP2 plant expression constructs.

Total RNA was isolated from Arabidopsis leaves (Logemann et al., 1987) and reverse transcribed from mRNA using a polyA primer mix (5'-A₃₀-C/G/T-3') and Superscript II (Invitrogen) according to a protocol of the supplier. Truncated Arabidopsis P2 cDNA fragments (At1g24280, 5' delta 195 bp termed cP2) were amplified from 1^{st} strand cDNA using primers ZM_S2 and ZM_S3 and PfuI DNA polymerase (Stratagene), and directly cloned into BamHI and SalI opened pBluescript SK vector (Stratagene) yielding pZM3. Similarly, BamHI and SalI digested cP2 fragments were introduced into the multiple cloning site of pA35 (plant expression cassette assembled in pUC18, see Höfte et al., 1991) between CaMV 35S promoter and OCS polyadenylation signal yielding pZM4. The entire plant expression cassette was transferred by complete HindIII and partial PvuII digest into HindIII and SnaBI opened vector pGSC1704 [HygR] (Plant Genetic Systems), yielding final binary construct pZM5 suited for Agrobacterium-mediated stable plant transformation (see Example 5 below).

Primers for amplification of Arabidopsis cP2 cDNA fragments:
**ZM_S2 sense** (BamHI recognition site underlined, start codon bold) (SEQ ID NO:1)
   5'-N₆-GGA TCC AAG **ATG** GTT GTC GTG CAA GAT GGA TCA GTA GCC ACC-3'
**ZM_A3 antisense** (SalI recognition site underlined, stop codon bold) (SEQ ID NO:2)
   5'-N₆-GTC GAC **TCA** CTG ATC AAG ACT TAG GTC TCC CCA TTG-3'

### Example 4: G6PDH activity test of the recombinant cP2 enzyme in E. coli.

For cloning into *E. coli* expression vector pET16b (Novagen) cP2 cDNA fragments were amplified from pZM4 (Fig. 3) using primers pET-cP2 sense and ZM_A3 (antisense, see above) and Phusion DNA polymerase (Finzymes). PCR products were digested with NcoI and SalI and cloned in *E. coli* XL1 blue (Stratagene) after ligation to NcoI-XhoI opened vector pET16b (Novagen), yielding pET-cP2. G6PDH activity of the cP2 enzyme was determined in a G6PDH-deficient *E. coli* strain. Host strain BL21(DE3) pLysS (Novagen) was modified by P1 transduction using *E. coli* zwf minus strain SU294 (Lee & Levy, 1992) resulting in BL21 ^{*G6PD*minus} *zwf*::Tn10[TetR] (Christian Schwöppe and Antje von Schaewen, unpublished). After retransformation, cP2 expression was induced in logarithmically growing BL21 ^{*G6PD*minus}: pET-cP2 cultures by adding IPTG (1 mM f.c) and allowed to grow for 2-3 h at 37°C. *E. coli* cells were harvested by centrifugation and adjusted to 10 OD₆₀₀. Extraction was in 100 mM NaH₂PO₄, 10 mM Tris-NaOH pH 8 supplemented with 0.1 mM Pefabloc SC and 0.02 mM NADP (to stabilize G6PDH) by 3 times sonication for 10 sec at 50 W (Branson sonifier). G6PDH activity of cP2 was characterized by K_{m[G6P]} = 0.58 mM, and K_{i[NADPH]} = 4.6 µM> K_{m[NADP+]} = 2.4 µM. The latter values differ slightly from those previously reported by Wakao & Benning (2005) for the recombinant enzyme with C-terminal Strep-tag (K_{i[NADPH]} = 22 µM > K_{m[NADP*]} = 17 µM).

Specific primer for cloning a pET16b-cP2 expression construct (without His-tag).
**pET-cP2 sense** (NcoI recognition site underlined, start codon bold) (SEQ ID NO:3)
   5'-NNNCC **ATG** GTT GTC GTG CAA GAT GGA TCA G TA G-3'

### Example 5: Generation of tobacco plants that overexpress cP2 (Atlg24280) in the cytosol.

Binary construct pZM5 (Example 4, see above) was directly transformed into Agrobacterium strain GV2260 (Deblaere et al., 1988) according to a protocol described by Höfgen & Willmitzer (1985). Generation of transgenic tobacco plants was by Agrobacterium cocultivation of *Nicotiana tabacum* var. Xanthi leaf discs with GV2260:pZM5 followed by a combined callus-shoot regeneration protocol as described by Voelker et al. (1987). Xanthi transformants were selected for high expression of cP2 in T0 using Northern-blot analyses, and by immunoblot analyses in T1 and all following generations (not shown) using a G6PDH antiserum specific for plant P2 isoenzymes (Wenderoth et al., 2000).

**Example 6:** Generation of Xanthi-cP2 plants with reduced levels of endogenous cytosolic G6PDH activity by supertransformation with a cyt*G6PD*-dsRNAi construct.

### Cloning of a cytG6PD-dsRNAi construct

The cyt*G6PD*-dsRNAi construct was designed based on tobacco cytG6PD isoforms (not shown). Approximately 400 bp were amplified by RT-PCR from total leaf RNA isolated from the *Nicotiana tabacum* variety Xanthi. The resulting fragment was inserted twice into vector pUC-RNAi (Chen et al., 2003) flanking the central first intron of potato GA20-Oxidase. The first insertion was via SalI/BamHI and the second insertion via XhoI/BgiII compatible ends.

Primers for cloning a tobacco G6PD-dsRNAi construct in pUC-RNAi (Chen et al., 2003)
**cyt*G6PD*-s** (sense, SalI site underlined) (SEQ ID NO:4)
   5'-CACCGTCGACAATATGAAGGCTATAAGGATGACC-3'
**cyt*G6PD*-as** (antisense, BamHI site underlined) (SEQ ID NO:5)
   5'-GGATCCTATATGACAGGTCTAATTCACTTTGAAC-3'

Then, the entire dsRNAi region was released by restriction digest with PstI and the expression cassette was inserted (between the strong CaMV 35S promoter and OCS polyadenylation signal) ofSdaI opened binary vector pBinAR[Kan] (Höfgen & Willmitzer, 1990).

### Supertransformation of Xanthi-cP2 lines using the binary cytG6PD-RNAi construct

Leaf discs of two independent T1 Xanthi-cP2 lines (weak cP2 83-1 and strong cP2 67-3) were transformed by the leaf-disc method (as in Example 5 above) using Agrobacterium strain GV2260 carrying the pBinAR-cytG6PD-RNAi construct. Regeneration of supertransformants was on double selective media containing 100 mg/l Kanamycin and 40 mg/l Hygromycin B.

### References

Chen S, Hofius D, Sonnewald U, Börnke F (2003) Temporal and spatial control of gene silencing in transgenic plants by inducible expression of double-stranded RNA. Plant J. 36: 731-740.
Colas V, Conrod S, Venard P, Keller H, Ricci P, Panabieres F (2001) Elicitin genes expressed in vitro by certain tobacco isolates of Phytophthora parasitica are down regulated during compatible interactions. Mol Plant Microbe Interact 14: 326-335.
Deblaere R, Bytebier B, De Greve H, Debroeck F, Schell J, van Montagu M, Leemans J (1985) Efficient octopine Ti plasmid-derived vectors of Agrobacterium-mediated gene transfer to plants. Nucl Acids Res 13: 4777-4788.
Fickenscher K, Scheibe R (1986) Purification and properties of the cytoplasmatic glucose-6-P dehydrogenase from pea leaves. Arch Biochem Biophys 247: 393-402.
Glaser BL, Brown DH (1955) Purification and properties of D-glucose 6-phosphate dehydrogenase. J Biol Chem 216: 67-79.
Höfgen R, Willmitzer L (1988) Storage of competent cells for Agrobacterium transformation. Nucl Acids Res 16: 9877.
Höfgen R, Willmitzer L (1990) Biochemical and genetic analysis of different patatin isoforms expressed in various organs of potato (Solanum tuberosum). Plant Sci 66: 221-230.
Höfte H, Faye L, Dickinson C, Herman EM, Chrispeels MJ (1991) The protein-body proteins phytohemagglutinin and tonoplast intrinsic protein are targeted to vacuoles in leaves of transgenic tobacco. Panta 184: 431-437.
Lee WT, Levy HR (1992) Lysine-21 of Leuconostoc mesenteroides glucose 6-phosphate dehydrogenase participates in substrate binding through charge-charge interaction. Protein Sci 1: 329-334.
Logemann J, Schell J, Willmitzer L (1987). Improved method for the isolation of RNA from plant tissues. Anal Biochem 163: 16-20.
Pugin A, Frachisse JM, Tavernier E, Bligny R, Gout E, Douce R, Guern J (1997) Early events induced by the elicitor Cryptogein in tobacco cells: Involvement of a plasma membrane NADPH Oxidase and activation of glycolysis and the pentose phosphate pathway. Plant Cell 9: 2077-2091.
Scharte J, Schön H, Weis E (2005) Photosynthesis and carbohydrate metabolism in tobacco leaves during an incompatible interaction with Phytophthora nicotianae. Plant, Cell Environ. 28: 1421-1435.
Thordal-Christensen H, Zhang ZG, Wei YD, Collinge DB (1997) Subcellular localization of H2O2 in plants: H2O2 accumulation in papillae and hypersensitive response during the barley-powdery mildew interaction. Plant J 11: 1187-1194.
Voelker T, Sturm A, Chrispeels MJ (1987) Differences in expression between two seed lectin alleles obtained from normal and lectin-deficient beans are maintained in transgenic tobacco. EMBO J 6: 3571-3577.
von Broembsen SL, Deacon JW (1996) Germination and further zoospore release from zoospore cysts of Phytophthora parasitica. Mycol Res 100: 1498-1504.
Way HM, Kazan K, Mitter N, Goulter KG, Birch RG, Manners JM (2002). Expression of the ShPAL phenylalanine ammonia lyase gene of Stylosanthes humilis in transgenic tobacco leads to enhanced disease resistance but impaired plant growth. Physiol Mol Plant Pathol 60: 275-282.
Wakao S, Benning C (2005) Genome-wide analysis of glucose-6-phosphate dehydrogenases in Arabidopsis. Plant J. 41: 243-256.
Wang R, Okamoto M, Xing X, Crawford NM (2003) Microarray analysis of the nitrate response in Arabidopsis roots and shoots reveals over 1,000 rapidly responding genes and new linkages to glucose, trehalose-6-phosphate, iron, and sulfate metabolism. Plant Physiol. 132: 556-567.
Wendt UK, Wenderoth I, Tegeler A, von Schaewen A (2000) Molecular characterization of a novel glucose-6-phosphate dehydrogenase from potato (Solanum tuberosum L.). Plant J. 23: 723-733.

## Claims

1. Method for producing eukaryotic organisms having enhanced pathogen resistance and/or resistance to stress, comprising the step of:
Expressing in the cytosol of said organism a glucose-6-phosphate dehydrogenase with increased NADPH tolerance compared to the endogenous cytosolic glucose-6-phosphate dehydrogenase.

2. Method according to claim 1, wherein the glucose-6-phosphate dehydrogenase with increased NADPH tolerance is an exogenous glucose-6-phosphate dehydrogenase.

3. Method according to claims 1 or 2, wherein the kinetic data of the glucose-6-phosphate dehydrogenase with increased NADPH tolerance fulfil the following relationships:
K_{i[NADPH]} > K_{m[NADP+]}, preferably K_{i[NADPH]} ≥ 2 x K_{m[NADP+]}, in particular K_{i[NADPH]} ≥ 5 x K_{m[NADP+]} and most preferred K_{i[NADPH]} ≥ 10 x K_{m[NADP+]}.

4. Method according to any one of claims 1 to 3, wherein the glucose-6-phosphate dehydrogenase with increased NADPH tolerance is a plastidic or peroxisomal glucose-6-phosphate dehydrogenase.

5. Method according to any one of claims 1 to 4, wherein the glucose-6-phosphate dehydrogenase with increased NADPH tolerance is significantly overexpressed, preferably to reach a concentration in the cytosol of at least twofold.

6. Method according to any one of claims 1 to 5, **characterized in that** it comprises the additional step of reducing, eliminating or suppressing the activity of the endogenous cytosolic glucose-6-phosphate dehydrogenase.

7. Method according to claim 6, wherein the activity of endogenous cytosolic glucose-6-phosphate dehydrogenase is reduced, eliminated or suppressed by targeted knock-out mutation or gene silencing, co-suppression, antisense or RNA interference.

8. Method according to any one of claims 1 to 7, wherein the eukaryotic organism is selected from plants, animals or fungi.

9. Method according to claim 8, wherein the plant is selected from higher plant species, preferably from Solanaceae, soy bean, maize, rice or Arabidopsis.

10. Method according to claims 8 or 9, wherein the plant is selected from tobacco, tomato, potato or pepper.

11. Eukaryotic transgenic organism having enhanced pathogen resistance and/or resistance to stress, **characterized in that** it expresses in its cytosol a glucose-6-phosphate dehydrogenase with increased NADPH tolerance compared to the endogenous cytosolic glucose-6-phosphate dehydrogenase.

12. Eukaryotic transgenic organism according to claim 11, wherein the kinetic data of the glucose-6-phosphate dehydrogenase with increased NADPH tolerance fulfil the following relationships: K_{i[NADPH]} > K_{m[NADP+]}, preferably K_{i[NADPH]} ≥ 2 x K_{m[NADP+]}, in particular K_{i[NADPH]} ≥ 5 x K_{m[NADP+]} and most preferred K_{i[NADPH]} ≥ 10 x K_{m[NADP+]}.

13. Eukaryotic transgenic organism according to claims 11 or 12, wherein the glucose-6-phosphate dehydrogenase with increased NADPH tolerance is an exogenous glucose-6-phosphate dehydrogenase and/or a plastidic or peroxisomal glucose-6-phosphate dehydrogenase.

14. Eukaryotic transgenic organism according to any one of claims 11 to 13, **characterized in that** the activity of its endogenous cytosolic glucose-6-phosphate dehydrogenase is reduced, eliminated or suppressed.

15. Eukaryotic transgenic organism according to any one of claims 11 to 14, **characterized in that** it is selected from plants, animals or fungi, and preferably is a higher plant species, in particular selected from the group consisting of Solanaceae, soy bean, maize, rice or Arabidopsis, particularly preferred a plant selected from tobacco, tomato, potato or pepper.
